(19) Europäisches Patentamt
European Patent Office
Office européen des brevets

(11) Veröffentlichungsnummer: **0 011 169**
A1

(12) # EUROPÄISCHE PATENTANMELDUNG

(21) Anmeldenummer: 79104177.5

(22) Anmeldetag: 29.10.79

(51) Int. Cl.³: **C 07 C 11/12**
C 07 C 11/21, C 07 C 13/00
C 07 C 13/11, C 07 C 13/19
C 07 C 15/42, C 07 C 33/035
C 07 C 33/05, C 07 C 33/28
C 07 C 43/14, C 07 C 43/15

(30) Priorität: 02.11.78 DE 2847425

(43) Veröffentlichungstag der Anmeldung: 28.05.80 Patentblatt 80 11

(84) Benannte Vertragsstaaten: BE CH DE FR GB IT NL

(71) Anmelder: HOECHST AKTIENGESELLSCHAFT
Zentrale Patentabteilung Postfach 80 03 20
D-6230 Frankfurt/Main 80(DE)

(72) Erfinder: Bestmann, Hans Jürgen, Prof. Dr.
Spitzwegstrasse 31
D-8520 Erlangen(DE)

(72) Erfinder: Vostrowsky, Otto, Dr.
Schleifweg 17c
D-8521 Uttenreuth(DE)

(72) Erfinder: Süss, Joachim
Flössaustrasse 162
D-8510 Fürth(DE)

(54) **Verfahren zur stereoselektiven Herstellung von (E),(E)-Di-olefinen.**

(57) Verfahren zur stereoselektiven Herstellung von (E), (E)-Diolefinen der Formel

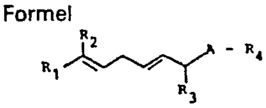

worin $R_1$ H, Alkyl, Alkenyl, Cycloalkyl, Cycloalkylalkyl, Aralkyl oder Aryl, A eine chemische Bindung oder einen gesättigten oder ungesättigten aliphatischen KW-Rest mit 1-10 C-Atomen, $R_4$ H, $-C_6H_5$, $-CH(R_5)OH$, $-CH(R_5)O$-Alkyl,

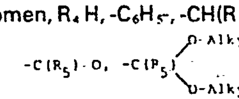 oder 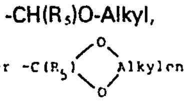

und $R_2$, $R_3$ und $R_5$ H oder Alkyl bedeuten durch aufeinanderfolgende Behandlung von Verbindungen der Formel

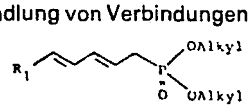

mit wasserfreien Basen und Verbindungen der Formel Hal-$CH(R_3)$-A-$R_4'$, worin $R_4'$ H, $-C_6H_5$, $-CH(R_5)$-O-Alkyl,

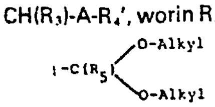 oder 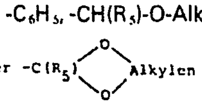 bedeutet und

anschließendes Behandeln der erhaltenen Zwischenprodukte mit $LiAlH_4$ und ggf. Säuren.

EP 0 011 169 A1

BAD ORIGINAL

Croydon Printing Company Ltd.

0011169

HOECHST AKTIENGESELLSCHAFT HOE 78/F 236 Dr.TG/sch

Verfahren zur stereoselektiven Herstellung von (E), (E)-Di-olefinen

Gegenstand der vorliegenden Erfindung ist ein Verfahren zur Herstellung von Verbindungen der Formel

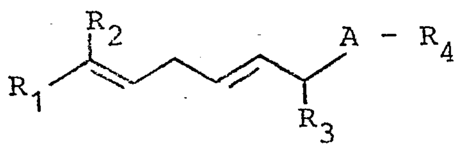

(I)

worin

$R_1$ Wasserstoff oder einen Alkyl-, Alkenyl- Cycloalkyl-, Cycloalkylalkyl-, Aralkyl- oder Arylrest mit bis zu 16 C-Atomen,

$R_2$ und $R_3$ H oder $(C_1-C_4)$Alkyl,

A eine chemische Bindung oder einen gesättigten oder ein- bis zweifach ungesättigten, geradkettigen oder verzweigten Kohlenwasserstoffrest mit 1 - 10 C-Atomen,

$R_4$ H, $-C_6H_5$, $-CH(R_5)OH$, $-CH(R_5)-O-(C_1-C_6)$Alkyl, $-C(R_5)=O$,

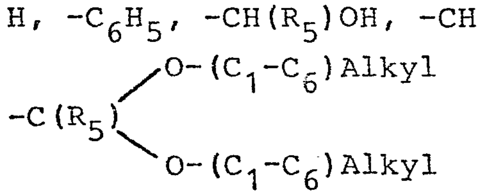 oder 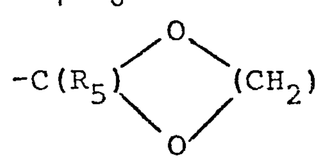 sowie

$R_5$ H oder $(C_1-C_4)$Alkyl bedeuten,

dadurch gekennzeichnet, daß man Verbindungen der Formel

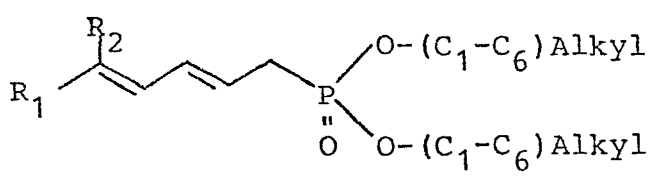

(II)

nacheinander mit starken wasserfreien Basen und einer Verbindung der Formel

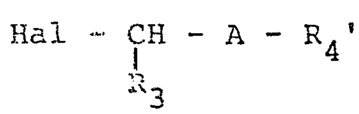

(III)

BAD ORIGINAL

worin "Hal" Chlor, Brom oder Jod und $R_4$' H, $-C_6H_5$,

$-CH(R_5)O-(C_1-C_6)$Alkyl, 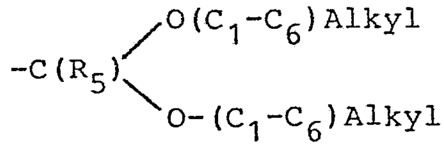 oder

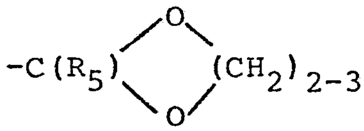

bedeutet, umsetzt und die erhaltenen Verbindungen der Formel

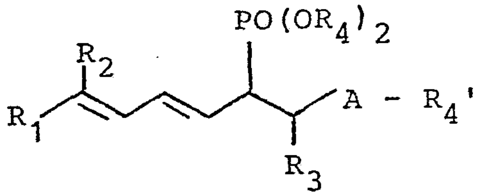 (IV)

mittels $LiAlH_4$ und gegebenenfalls anschließendes Behandeln mit Säuren in Verbindungen der Formel I überführt.

Überraschenderweise erfolgt bei dem letzten Verfahrensschritt (IV → I) eine Umlagerung der einen Doppelbindung unter Ausbildung einer Konfiguration, bei der zwei Doppelbindungen durch eine $-CH_2$-Gruppe voneinander getrennt sind. Da konjugierte Doppelbindungen im allgemeinen gegenüber isolierten Doppelbindungen energetisch bevorzugt sind, war dieser Reaktionsverlauf nicht zu erwarten. Zudem ist die Umsetzung in hohem Maße stereoselektiv und führt zu zweifach ungesättigten Di-olefinen mit einem konstanten Anteil von ca. 85 % an (E), (E)-Isomeren.

Die Ausgangsstoffe der Formel II lassen sich in bekannter Weise aus Halogeniden der Formel

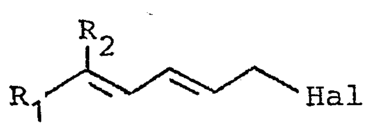 (V)

und Trialkylphosphiten unter den Verfahrensbedingungen der Arbusow-Reaktion erhalten. Die weitere Umsetzung derselben mit starken wasserfreien Basen - als solche kommen z.B. Butyllithium, Phenyllithium, Natriumamid oder Na-hexamethyldisilazan in Frage - bei niedrigen Temperaturen führt zunächst zu einem Anion, das anschließend mit Verbindungen der Formel III zu IV umgesetzt wird. Für die beiden Teil-

BAD ORIGINAL

schritte der Umsetzung II $\rightarrow$ IV verwendet man organische aprotische Lösungsmittel wie Benzol, Toluol, Dioxan, Tetrahydrofuran (THF) oder Diäthyläther. Die Reaktionstemperaturen liegen im Bereich zwischen -80° und Raumtemperatur, vorzugsweise arbeitet man bei Temperaturen um -60°C.

Will man zu Verbindungen der Formel I gelangen, in denen $R_2$ eine Gruppe der Formel $-CH(R_3)-OH$ oder $-C(R_3)O$ darstellt, so muß man in den Ausgangsprodukten der Formel III die entsprechenden Reste durch Schutzgruppen blockieren, um Nebenreaktionen zu vermeiden. Zum Schutz der Gruppe $-CH(R_3)OH$ dient die Verätherung mit Gruppen, die sich anschließend nach literaturbekannten Methoden - z.B. durch Behandlung mit starken Säuren - wieder abspalten lassen. Besonders geeignet wegen ihrer leichten Spaltbarkeit sind hierfür die Tetrahydropyranyläther. Die Aldehyd- oder Ketofunktion kann durch Acetalisierung bzw. Ketalisierung mittels ein- oder zweiwertiger Alkohole geschützt werden.

In den erhaltenen Verbindungen der Formel IV wird anschließend mit Lithiumaluminiumhydrid bei Temperaturen von -20°C bis Raumtemperatur (+25°C) der Phosphonesterrest wieder abgespalten; dabei lagert sich die dem Phosphoratom näherliegende Doppelbindung um. Für diese Reaktion werden die für Umsetzungen mit $LiAlH_4$ üblichen wasserfreien Lösungsmittel verwendet; bevorzugt nimmt man Äther wie Diäthyläther, Dioxan oder THF. Falls die Verbindung IV in $R_4$'-Stellung eine mittels Schutzgruppen blockierte Äther-, Aldehyd- oder Ketofunktion aufweist, wird die Schutzgruppe gewünschtenfalls anschließend auf üblichem Wege wieder abgespalten. Die für die Bildung und anschließende Spaltung von Schutzgruppen der Alkohol-, Aldehyd- und Ketofunktion üblichen Methoden sind im einzelnen ausführlich in "Compendium of Organic Synthetic Methods", Bd. 1, S. 124 ff. 174 ff. und 449 ff., bzw. Bd. 3, S. 56 ff., 85 ff. und 291 ff. beschrieben. Schließlich wird das Endprodukt aus dem Reaktionsgemisch mit einem organischen Lösungsmittel

wie Äther extrahiert, die organische Lösung getrocknet und das Lösungsmittel abdestilliert.

Die erfindungsgemäß erhaltenen Verbindungen können teilweise als Bausteine zum Aufbau von Naturstoffen verwendet werden; zum Teil wirken sie als Pheromone oder können - z.B. durch Acetylierung einer in $R_4$-Stellung vorhandenen Hydroxylgruppe - in Verbindungen mit Pheromonwirkung überführt werden.

Herstellungsbeispiele:

1) (E)-7, (E)-10-Dodecadien-1-ol

a) Zu 16.6 g (100 mmol) Triethylphosphit läßt man bei 110° langsam 16.1 g (100 mmol) (E)-2, (E)-4-Hexadienylbromid zutropfen. Danach wird die Mischung 4 Stunden auf 150 - 160°C erhitzt und destilliert. Man erhält 15.9 g (73 %) Diethyl-(E)-2, (E)-4-hexadienylphosphonat vom Sdp. 82-84° / 0.013 mbar.

b) Unter Stickstoffatmosphäre und Rühren läßt man zu einer Lösung von 4.36 g (20 mmol) des Phosphonats gemäß a) in 40 ml THF 24 mmol einer Butyllithium-Lösung bei -60°C zutropfen und rührt anschließend noch 1 Stunde bei Raumtemperatur. Die rote Lösung kühlt man erneut auf -60°C ab, gibt tropfenweise 5.3 g (20 mmol) 6-(Tetrahydro-2-pyranyloxy)-hexylbromid hinzu und läßt nach weiteren 30 min. bei -60°C die Lösung über Nacht auf Raumtemperatur erwärmen. Das Reaktionsgemisch wird mit $NH_4Cl$-Lösung versetzt, mit Ether extrahiert und die vereinigte Etherphasen getrocknet. Das Addukt wird schließlich über eine Kieselgelsäule gereinigt. (Rohausbeute 82 %)

c) 4.03 g (10 mmol) des alkylierten Phosphonats (Rohprodukt) löst man in 50 ml wasserfreiem Ether und versetzt unter Eiskühlung mit 0.23 g (6 mmol) $LiAlH_4$. Nach 1 Stunde

Rühren bei 0° und 1 Stunde bei Raumtemperatur werden 2 ml Eiswasser zugetropft, das Gemisch über Kieselgur filtriert und der Filterkuchen mit Ether nachgewaschen. Das Filtrat wird am Rotationsverdampfer eingedampft, der Rückstand mit 40 ml wäßrigem Methanol aufgenommen, mit 0.1 g p-Toluolsulfonsäure versetzt und 2 Stunden unter Rückfluß erhitzt. Nach dem Abkühlen wird das Methanol abdestilliert und die wäßrige Phase einige Male mit Ether extrahiert. Die vereinigten Etherphasen werden mit $NaHCO_3$-Lösung gewaschen, über $MgSO_4$ getrocknet und destilliert. Man erhält 0.84 g (46 %) (E)-7, (E)-10-Dodekadien-1-ol vom Sdp. 85-90° / 0.013 mbar (Badtemperatur).

2) In analoger Weise erhält man aus 4.36 g (20 mmol) Phosphonat (gemäß 1a) und 5.86 g (20 mmol) 8-(Tetrahydro-2-pyranyloxy)octylbromid 1.01 g (48 %) (E)-9, (E)-12-Tetradecadien-1-ol, Sdp. 105-110° / 0.04 mbar (Badtemperatur).

3) In analoger Weise erhält man aus 4.36 g (20 mmol) Phosphonat (1a) und 6.42 g (20 mmol) 10-(Tetrahydro-2-pyranyloxy)-decylbromid 1.07 g (45 %) (E)-11, (E)-14-Hexadecadien-1-ol, Sdp. 115-120° / 0.04 mbar (Badtemperatur).

4) Gemäß 1a) wird aus 16.6 g (100 mmol) Triethylphosphit und 18.9 g (100 mmol) (E)-2, (E)-4-Octadienylbromid, Diethyl-(E)-2, (E)-4-octadienylphosphonat dargestellt. Ausb. 9.85 g (40 %), Sdp. 93° / 0.013 mbar.

In analoger Weise wie bei Herstellungsbeispiel 1 erhält man aus 6.15 g (25 mmol) Phosphonat und 6.63 g (25 mmol) 6-(Tetrahydro-2-pyranyloxy)-hexylbromid 2.15 g (41 %) (E)-7, (E)-10-Tetradecadien-1-ol, Sdp. 105-110° / 0.013 mbar (Badtemperatur).

5) Auf gleiche Weise erhält man wie bei Beispiel 4 aus 6.15 g Phosphonat und 7.33 g (25 mmol) 8-(Tetrahydro-2-pyranyloxy)-octylbromid 2.38 g (40 %) (E)-9, (E)-12-Hexadecadien-1-ol, Sdp. 115-120° / 0.013 mbar (Badtemperatur).

6) Aus 8.3 g (50 mmol) Triethylphosphit und 12.15 g (50 mmol) 5,9-Dimethyldeca-2,4,8-trienylbromid (aus käuflichem Citral mit einem Isomerenverhältnis E/Z = 6:4) erhält man wie bei 1a) 4.20 g (28 %) Diethyl-(5,9-dimethyldeca-2,4,8-trienyl)-phosphonat, Sdp. 130-140° / 0.013 mbar (Badtemperatur).
Aus 3.00 g (10 mmol) des Phosphonats und 2.17 g (10 mmol) Geranylbromid 1.68 g (56 %) erhält man gemäß Beispiel 1 1,2-Digeranylethylen = 2,6,13,17-Tetramethyloctadeca-2,6,9,12,16-pentaen, Sdp. 110-120° / 0.026 mbar (Badtemperatur)

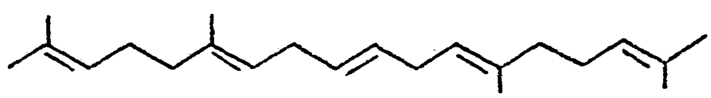

Aufgrund des Isomerenverhältnisses der Ausgangsverbindung Citral (E/Z = 6:4) enthielt das Syntheseprodukt auch 40 % (Z)-Isomeres an der 6-Doppelbindung.

7) Analog Beispiel (6) erhält man aus 1.5 g (5 mmol) des Phosphonats und 0.885 g (5 mmol) Cyclohexylmethylbromid 0.754 g (58 %) 11-Cyclohexyl-2,6-dimethylundeca-2,6,9-trien, Sdp. 120° /0.015 mbar (Badtemperatur).

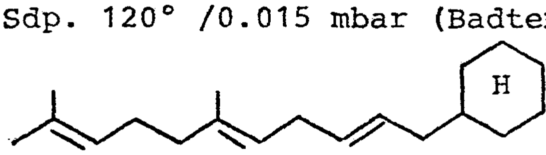

Zum Isomerenverhältnis vergleiche Beispiel 6.

8) Analog Beispiel 6) erhält man aus 1.5 g (5 mmol) des Phosphonats und 0.615 g (65 mmol) Isopropylbromid 0.567 g (55 %) 2,6,11-Trimethyldodeca-2,6,9-trien, Sdp. 50-55° / 0.013 mbar (Badtemperatur).

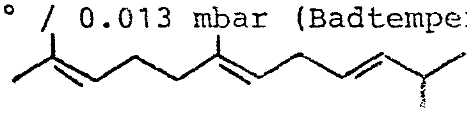

zum Isomerenverhältnis vergleiche Beispiel 6.

9 a) Zu 16.6g (100 mmol) Triethylphosphit tropft man bei 110°C langsam eine Lösung von 22.3g (100 mmol) 5-Phenyl-(E)-2, (E)-4-pentadienylbromid in 50 ml abs. Xylol. Das Reaktionsgemisch wird danach 4 h auf 150-160°C erhitzt, anschließend das Lösungsmittel abdestilliert und der Rückstand aus Ether umkristallisiert. Man erhält 16.8g (60 %) Diethyl-5-phenyl-(E)-2, (E)-4-pentadienylphosphonat, Smp. 71-72°C.

b) In analoger Weise wie bei Beispiel 1) erhält man aus 3.5 g (12.5 mmol) des Phosphonats 9a) und 2.14g (12.5 mmol) Benzylbromid 1.70 g (58 %) 1,6-Diphenyl-(E)-1, (E)-4-hexadien, Sdp. 115-118°C / 0.013 mbar (Badtemperatur).

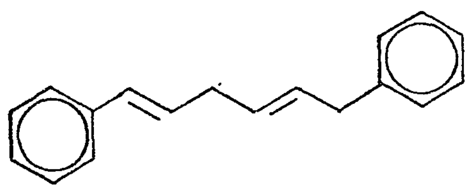

10) Aus 2.8 g (10 mmol) des Phosphonats 9a) und 2.23g (10 mmol) 5-Phenyl-(E)-2, (E)-4-pentadienylbromid erhält man in analoger Weise 1.37 g (48 %), 1,10-Diphenyl-(E)-1, (E)-3, (E)-6, (E)-9-decatetraen, Smp. 65-66°C (aus Methanol umkristallisiert).

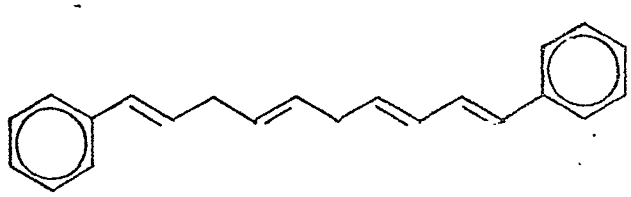

11) Aus 7.0 g (25 mmol) des Phosphonats 9a) und 6.63 g (25 mmol) 6-(Tetrahydro-2-pyranyloxy)-hexylbromid erhält man nach dem gleichen Verfahren 2.5 g (41 %) 11-Phenyl-(E)-7, (E)-10-undecadien-1-ol, Sdp. 150-155°/ 0.013 mbar (Badtemperatur).

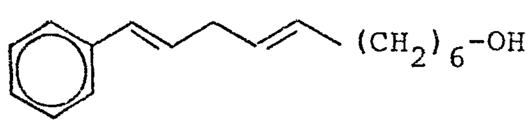

12 a) Gemäß Beispiel 1a) wird aus 8.3 g (50 mmol) Triethylphosphit und 7.35 g (50 mmol) (E)-2,4-Pentadienylbromid Diethyl-(E)-2,4-pentadienylphosphonat dargestellt. Ausb. 6.95 g (68 %), Sdp. 60-65°C / 0.013 mbar (Badtemperatur).

b) Auf gleiche Weise wie in Beispiel 1) erhält man aus 3.06 g (15 mmol) des Phosphonats 12a) und 4.19 g (15 mmol) 7-(Tetrahydro-2-pyranyloxy)-heptylbromid 1.0 g (37 %) (E)-8,11-Dodecadien-1-ol, Sdp. 70-75°C/ 0.013 mbar (Badtemperatur).

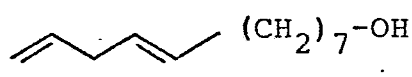

PATENTANSPRUCH

Verfahren zur Herstellung von Verbindungen der Formel

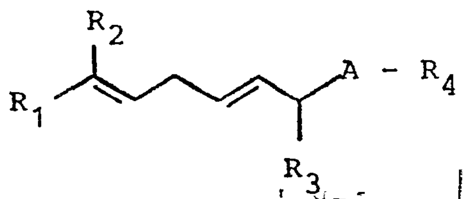

(I)

worin

$R_1$ Wasserstoff oder einen Alkyl-, Alkenyl-, Cycloalkyl-, Cycloalkylalkyl-, Aralkyl- oder Arylrest mit bis zu 16 C-Atomen,

$R_2$ und $R_3$ H oder $(C_1-C_4)$Alkyl,

A eine chemische Bindung oder einen gesättigten oder ein- bis zweifach ungesättigten, geradkettigen oder verzweigten Kohlenwasserstoffrest mit 1-10 C-Atomen,

$R_4$ H, $-C_6H_5$, $-CH(R_5)OH$, $-CH(R_5)-O-(C_1-C_6)$Alkyl,

$-C(R_5)=O$, 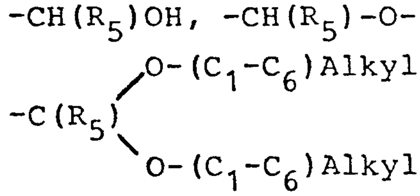 oder 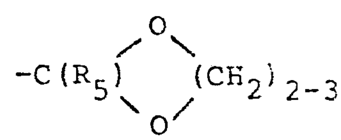

sowie

$R_5$ H oder $(C_1-C_4)$Alkyl bedeuten,

dadurch gekennzeichnet, daß man Verbindungen der Formel

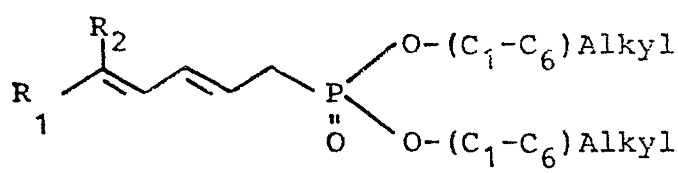

(II)

nacheinander mit starken wasserfreien Basen und einer Verbindung der Formel

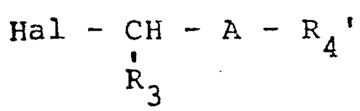

(III)

worin "Hal" Chlor, Brom oder Jod und $R_4'$ H, $-C_6H_5$, $-CH(R_5)O-(C_1-C_6)$Alkyl,

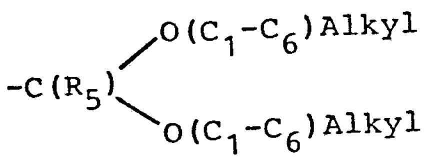

oder

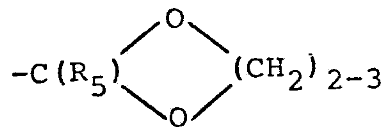

bedeutet, umsetzt und die erhaltenen Verbindungen der Formel

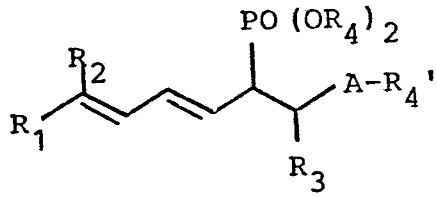

mittels $LiAlH_4$ und gegebenenfalls anschließendes Behandeln mit Säuren in Verbindungen der Formel I überführt.

Europäisches Patentamt

# EUROPÄISCHER RECHERCHENBERICHT

0011169

Nummer der Anmeldung

EP 79 104 177.5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A1 - 2 542 601 (HOFFMANN-LA ROCHE)<br>* Anspruch 1 *<br>-- | 1 |
| A | GB - A - 1 150 848 (TOYO RAYON)<br>* Ansprüche 1 und 7 *<br>-- | 1 |
| A | US - A - 4 028 429 (R.D. CRAMER)<br>* Anspruch 1 *<br>-- | 1 |
| | TETRAHEDRON LETTERS, Band 1977, Nr. 13, Oxford, New York, Paris, Frankfurt<br>B. DESCHAMPS et al. "Stereoselectivity of wittig type olefin synthesis using 5 membered cyclic phosphine oxides or phosphonous acid dimethyl-amides"<br>Seiten 1137 bis 1140<br>-- | 1 |
| A | TETRAHEDRON LETTERS, Band 1977,Nr. 13, Oxford, New York, Paris, Frankfurt<br>E. BREUER et al. "Stereoselectivity of wittig type olefin synthesis using five-membered cyclic phosphonates, preferential formation of cis olefins"<br>Seiten 1141 bis 1144<br>-- | 1 |

./..

X Der vorliegende Recherchenbericht wurde für alle Patentansprüche erstellt.

KLASSIFIKATION DER ANMELDUNG (Int.Cl.3)

C 07 C 11/12
C 07 C 11/21
C 07 C 13/00
C 07 C 13/11
C 07 C 13/19
C 07 C 15/42
C 07 C 33/035
C 07 C 33/05
C 07 C 33/28
C 07 C 43/14
C 07 C 43/15
C 07 C 43/303
C 07 C 47/21

RECHERCHIERTE SACHGEBIETE (Int. Cl.3)

C 07 C 11/12
C 07 C 11/21
C 07 C 13/00
C 07 C 13/11
C 07 C 13/19
C 07 C 15/42
C 07 C 33/035
C 07 C 33/05
C 07 C 33/28
C 07 C 43/14
C 07 C 43/15
C 07 C 43/303
C 07 C 47/21

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 13-02-1980 | KNAACK |

Europäisches Patentamt

EUROPÄISCHER RECHERCHENBERICHT

Nummer der Anmeldung

EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG |
|---|---|---|---|
| A | TETRAHEDRON LETTERS, Band 1978, Nr. 30, Oxford, New York, Paris, Frankfurt G.L. v. MOURIK et al. "Stereo- and regiospecific synthesis of unbranched alkenes and 1,4-alkadienes by means of stabilised organocuprates" Seiten 2705 bis 2708 ---- | 1 | C 07 C 49/203<br>C 07 C 49/217<br>C 07 D 317/12<br>C 07 D 319/06 |

RECHERCHIERTE SACHGEBIETE (Int. Cl.3)

C 07 C 49/203
C 07 C 49/217
C 07 D 317/12
C 07 D 319/06

EPA Form 1503.2 06.78

BAD ORIGINAL